Europäisches Patentamt

European Patent Office

Office européen des brevets

(19)

(11) Publication number: **0 180 983**
**B1**

(12) ## EUROPEAN PATENT SPECIFICATION

(45) Date of publication of patent specification: **03.08.88**

(21) Application number: **85114146.5**

(22) Date of filing: **06.11.85**

(51) Int. Cl.⁴: **C 07 C 85/06,** C 07 C 87/10 //
B01J29/06

(54) **Amines via the amination of alkanols using dealuminated zeolites.**

(30) Priority: **07.11.84 US 669286**

(43) Date of publication of application:
**14.05.86 Bulletin 86/20**

(45) Publication of the grant of the patent:
**03.08.88 Bulletin 88/31**

(84) Designated Contracting States:
**BE DE FR GB NL**

(56) References cited:
**FR-A-2 451 909**
**US-A-3 506 400**
**US-A-4 082 805**
**US-A-4 434 300**
**US-A-4 458 092**

**PATENT ABSTRACTS OF JAPAN; unexamined applications, field C, vol. 7, no. 135, June 11, 1983 THE PATENT OFFICE JAPANESE GOVERNMENT PAGE 88 C 170**

(73) Proprietor: **AIR PRODUCTS AND CHEMICALS, INC.**
**Route no. 222**
**Trexlertown Pennsylvania 18087 (US)**

(72) Inventor: **Deeba, Michel**
**1243 N. 22nd Street**
**Allentown, PA 18104 (US)**

(74) Representative: **Dipl.-Ing. Schwabe, Dr. Dr. Sandmair, Dr. Marx**
**Stuntzstrasse 16**
**D-8000 München 80 (DE)**

Courier Press, Leamington Spa, England.

EP 0 180 983 B1

**Description**

Technical field

This invention relates to a catalytic process for producing amines by the reaction of ammonia or ammonia type compound with $C_{2-8}$ alkanol over partially dealuminated zeolites.

Background of the prior art

The catalyzed reaction of ethanol and ammonia to produce the mono-, di-, and triethylamines is well known in the art. Presently, the ethylamines are produced commercially by a continuous process by reacting ethanol and ammonia in the presence of an amorphous silica-alumina catalyst. This continuous process yields an equilibrium controlled distribution of the ethylamines.

U.S. Patent 3,384,667 discloses a method for producing monosubstituted and disubstituted amines in preference to trisubstituted amines by reacting ammonia with an alcohol in the presence of a crystalline metal aluminosilicate catalyst having pores of a diameter that pass the monosubstituted and disubstituted amine products but are too small to pass the trisubstituted amine product. Hydrogen exchanged crystalline alumino-silicates are defined as crystalline metal alumino-silicates in the patent.

US—A—4,082,805 discloses a process for the selective production of primary aliphatic amines by reaction of a $C_1$—$C_5$ alcohol or ether with ammonia in the presence of a catalyst comprising a crystalline aluminosilicate having the structure of ZSM-5, ZSM-11 or ZSM-21, at 300 to 500°C and at $10^5$ to $6,89 \times 10^6$ Pa (one atmosphere to 1000 psig) pressure, the feed rate of alcohol or ether and ammonia being within the ratio of 1:1 to 5:1 g/hr.

US—A—4,191,709 discloses a process for the manufacture of amines by reacting an alcohol with ammonia in the presence of the hydrogen form of zeolite FU-1 or zeolite FU-1 in which some or all of the protons have been replaced by bivalent or trivalent cations. The related US—A—4,205,012 is similar except that the catalyst comprises zeolite FU-1 in which some or all of the protons have been replaced by monovalent cations, for example, sodium.

US—A—4,229,374 discloses a process for producing tertiary amines by reacting alcohols with ammonia, primary amines or secondary amines in the presence of a specific catalyst. The catalyst comprises a mixture of copper, tin and an alkali metal supported on a suitable carrier, such as artificial and natural zeolites.

US—A—4,254,061 discloses a process for producing monomethylamine by reacting methanol and ammonia, in such amounts so as to provide a C/N ratio, from the methanol and ammonia reactants, of 0.5—1.5, over a catalyst which is (a) mordenite wherein the primary cation is Li, Na HNa having at least 2% Na by weight, K, Ca, Sr, Ba, Ce, Zn or Cr; (b) ferrierite wherein the primary metal cation is Li, Na, K, Ca, Sr, Ba, Ce or Fe; (c) erionite ore; (d) calcium erionite; or (e) clinoptilolite ore.

US—A—4,436,938 discloses a process for producing methylamines by reacting methanol or dimethyl ether and ammonia over a binderless zeolite 5A catalyst. The use of a binderless zeolite enhances selectivity to dimethylamine as opposed to mono or trimethylamine.

FR—A—2,451,909 discloses a process for producing methylamines, by the reaction of methanol with ammonia using an acid activated montmorillonite as the catalyst.

Summary of the invention

This invention relates to an improved process for the preparation of alkyl amines, by the vapor phase catalytic reaction of an alkanol having from 2 to 8 carbon atoms or $C_{2-6}$ alkyl ether with ammonia, a primary or secondary amine. In the basic process the $C_{2-8}$ alkanol or $C_{2-6}$ alkyl ether is reacted at a temperature and pressure sufficient to effect formation of the amine the improvement generally resides in the use of a partially dealuminated crystalline alumino-silicate zeolite of the molecular sieve type as the catalyst.

There are several advantages-associated with the invention as compared to prior art processes. These advantages include:

greater catalytic activity than is achieved with nondealuminated zeolite catalysts particularly in the formtion of ethylamines;

better activity than H—Y and rare-earth-Y zeolite at the higher temperature where the reaction becomes diffusion limited;

an ability to form a variety of alkyl amines at very high rates of alcohol conversion with significantly less olefin make than other strong acidic Y zeolites;

an ability to form in high selectivity mono- and diethyl amine with substantially complete elimination of the trisubstituted amine; and

outstanding catalyst life as in the case of Y zeolite, presumably caused by improved zeolite stability.

Detailed description of the invention

The alkanols or ethers suited for the invention include the $C_{2-8}$ alkanols and $C_{2-6}$ alkylethers. Examples of alkanols or ethers suited for producing amines by this process are ethanol, propanol, butanol, isobutanol, hexanol and cyclohexanol. Of these ethanol is preferred in practicing the invention. Alkylethers can also be aminated and these include diethylether, dimethylether, etc.

In the amination of the alkanols or ether an ammonia or an ammonia type compound, i.e. a primary or secondary amine is used as the co-reactant. If one uses a primary or secondary amine as the reactant, secondary and tertiary amines are formed as product. Suitable primary and secondary amines can be represented by the formulas $RNH_2$ and $(R)_2NH$ where each R is a hydrocarbon group having from 1—6 carbon atoms in the structure. Examples of amines include methylamine, dimethylamine, and ethylamines.

In the process, an ammonia type compound is reacted with the alkanol or ether at temperatures broadly from 150—500°C, but preferably at temperatures of 350—450°C. Lower temperatures result in lower conversion and higher temperatures usually result in lower selectivity.

Pressures suited for practicing the invention are from $6,89\times10^5$ to $4,13\times10^7$ Pa (100—6,000 psig) with preferred pressures of from $6,89\times10^5$ to $1,37\times10^7$ Pa (100 to 2,000 psig).

Another important variable in the gas phase amination of the alkanol is the mole ratio of ammonia to alkanol in the feed. Generally, the mole ratio can be from 0.2—20 moles ammonia per mole of alkanol with preferred ranges of from about 1—10 to 1. Mole ratios higher than 10:1 of course require greater energy expenditure to handle the larger quantities of gas that pass through the reactor, and there seems to be no significant advantage in terms of increased selectivity or conversion at these higher ratios of ammonia to alkanol.

The gas hourly space velocity (GHSV) which has units of volume of gas per volume of catalyst in unit time i.e. (cc gas at STP)/(cc catalyst hours$^{-1}$). The gas hour space velocity can be from 500—15,000 with a preferred range being from 1,000—12,000. As the space velocity is increased toward the upper end of the range, conversion generally falls dramatically, particularly above a space velocity of about 12,000.

The catalyst utilized in the amination process of this invention is commonly referred to as a partially dealuminated zeolite. Enhanced conversions are normally noted with those zeolitic materials which are in the acid form, e.g., the hydrogen ion or rare earth metal ion exchanged zeolites. Representative examples of zeolites suited for use in the acid form include Y, X, clinoptilolite, offretite, erionite, mordenite, montmorillonite and the ZSM family.

The dealumination of the zeolitic materials has been found to enhance the rate of conversion of the alkanol or ether to amine without interfering with the selectivity of the catalyst system. Partial dealumination also improves catalyst life presumably because stability of the zeolite is improved by increasing the silica to alumina ratio. It also improves conversion presumably by reducing the resistance reactant diffusion within the zeolite pores. By dealumination it is meant that the zeolite is treated with a dealuminating agent such as a chelating agent or acid for the purpose of removing aluminum atoms from the zeolite structure. Typically the percent aluminum removed is sufficient to effect removal of 10—90% from the total alumina ($Al_2O_3$ by weight) present in the zeolite material. For example, mordenite normally has an Si to Al molar ratio of about 5:1. The Si to Al molar ratio for partially dealuminated mordenite should be from 6 to 100:1 with preferred molar ratios of 6 to 20:1. The silicon to aluminum molar ratio of a Y zeolite is about 2.2:1. The Si to Al ratio for a partially dealuminated Y zeolite should be from 2.5 to 50:1 with a preferred Si to Al molar ratio of 2.5 to 15:1.

There are several techniques available for removing aluminum atoms from the structure and some involve the treatment of the zeolite material with a chelating agent such as acetyl acetonate, ethylenediamine-tetracetic acid, or by treatment with a mineral acid, e.g. hydrochloric acid or by activation using silicon tetrachloride. Aluminum atom removal is effective at temperatures from about 25—100°C within a time frame of 1 to 24 hours followed by calcination at temperatures of about 400°C. Examples of various techniques for removing aluminum atoms from zeolite materials to produce catalytic compositions are noted in U.S. 3,506,400; U.S. 3,937,791; and U.S. 3,761,396; these techniques are incorporated by reference.

The following examples are provided to illustrate various embodiments to the invention.

Example 1
Preparation of dealuminated H-mordenite

A 60—80 gram sample of 1/16″ extrudate of H-mordenite sold by the Norton Company under designation Z-900H was dealuminated by adding this catalyst to 2 liters of distilled water containing from 5—50 milliliters of 12 normal aqueous hydrochloric acid and 1 molar ammonium chloride. The solution was refluxed for 2 hours, washed with hot water until no chloride ions were detected and then dried at 150°C. The dealumination procedure was then repeated to achieve the desired degree of dealumination. The Si to Al molar ratio was about 6:1 and higher. The X-ray analysis of the dealuminated zeolite showed no loss of crystallinity. The materials tested had a Si to Al molar ratios of 6 and 8 to 1.

Example 2
A series of runs was made to produce ethylamines. Ammonia and ethanol in such amounts so as to provide an ammonia: ethanol molar ratio of about 2:1 were passed over about 5.34 grams of catalyst in a Berty recycle reactor (a fixed bed reactor with a large (greater than 20) internal recycle ratio). Under these conditions the Berty reactor is gradientless and behaves like a continually stirred torque reactor (CSTR). Rates of reaction can be calculated directly as moles converted per gram of catalyst per second of residence time.

The reaction was performed at a total pressure of $1,8\times10^6$ Pa (18 atmospheres) at a gas hourly space velocity of 9300 and at a variety of temperatures from 550 to 750°F (288 to 399°C). The reactor feeds and

3

**0 180 983**

effluent were analyzed by an on-line calibrated gas chromatograph. The streams were maintained gaseous from the reactor to the chromatograph sampling valve.

The catalysts tested were amorphous silica-alumina, H-mordenite, REY zeolite, H—Y zeolite, H-erionite and partially dealuminated H-mordenite. The REY zeolite was Union-Carbide's SK-500 rare earth exchanged zeolite catalyst. The remaining hydrogen exchanged aluminosilicate catalyst, namely H—Y zeolite, H-erionite and H-mordenite were prepared according to conventional procedures for hydrogen exchange from Na Y zeolite, erionite ore and sodium mordenite, respectively.

The results are provided in Tables 1—5.

TABLE 1

Ethanol conversion rates as a function of temperature
(g mole $C_2H_5$ OH/g of catalyst sec)

| Catalyst | Temperature | | |
| | (550°F) | (650°F) | (750°F) |
| | (288°C) | (343°C) | (400°C) |
| --- | --- | --- | --- |
| 1. Silica-alumina (LA-30) | $0.36\times10^{-7}$ | $1.4\times10^{-6}$ | $4.6\times10^{-6}$ |
| 2. Rey Zeolite | $2.3\times10^{-7}$ | $3.4\times10^{-6}$ | $3.7\times10^{-6}$ |
| 3. H—Y Zeolite | $4.0\times10^{-7}$ | $3.4\times10^{-6}$ | $3.8\times10^{-6}$ |
| 4. H-Erionite | nil | $1.1\times10^{-6}$ | $2.6\times10^{-6}$ |
| 5. H-Mordenite | $0.4\times10^{-7}$ | $0.8\times10^{-6}$ | $1.9\times10^{-6}$ |
| 6. Partially dealuminated H-Mordenite (Ex. 1) | $2.0\times10^{-7}$ | $2.0\times10^{-6}$ | $4.2\times10^{-6}$ |
| 7. Partially Dealuminated H-Mordenite (Ex. 2) (1/8″) | $2.0\times10^{-7}$ | $2.8\times10^{-6}$ | $5.3\times10^{-6}$ |

Table 1 shows that dealuminization of H-mordenite enhanced the amination rate of ethanol significantly as compared to H-mordenite itself. (Compare runs 5—7).

TABLE 2

Comparison of ethanol conversion rates
Zeolite aluminosilicate catalyst/silica-alumina

| Catalyst | Temperature | | |
| | (550°F) | (650°F) | (750°F) |
| | (288°C) | (343°C) | (400°C) |
| --- | --- | --- | --- |
| 8. Silica-alumina | 1 | 1 | 1 |
| 9. REY | 6.5 | 2.5 | 0.8 |
| 10. H—Y | 11.0 | 2.5 | 0.8 |
| 11. H-Erionite | nil | 0.8 | 0.6 |
| 12. H-Mordenite | 1 | 0.6 | 0.5 |
| 13. Partially dealuminated Z-900H (Ex. 1) | 5.6 | 1.4 | 0.9 |
| 14. Partially dealuminated Z-900H (Ex. 2) | 5.6 | 2.0 | 1.2 |

Runs 8—14 compare the rate of ethanol amination of various zeolites to the rate of amination of ethanol obtained with silica-alumina. At lower temperatures (550 and 650°F, 288 and 343°C) zeolites were more effective than silica-alumina. Rare-earth-Y and H—Y zeolites were the most effective and showed

4

**0 180 983**

better rates of ethanol conversion than H-mordenite. But, at the higher temperature (750°F, 400°C) where reaction is diffusion limited dealuminated H-mordenite showed superior activity over zeolites and silica-alumina (compare Example 14 with 8—12).

Dealuminated H-mordenite, on the other hand, showed significant improvement over the commercially available H-mordenite (Z-900H) (Table 3).

TABLE 3
Rate of ethanol conversion
(Dealuminated H-mordenite/H-mordenite)

| | Temperature | | |
| --- | --- | --- | --- |
| | (550°F) | (650°F) | (750°F) |
| Catalyst | (288°C) | (343°C) | (400°C) |
| 15. H-Mordenite (Z-900H) | 1 | 1 | 1 |
| 16. Partially dealuminated Z-900H (Ex. 1) | 5.6 | 2.3 | 1.8 |
| 17. Partially dealuminated Z-900H (Ex. 2) | 5.6 | 3.3 | 2.3 |

The partially dealuminated H-mordenite showed significantly higher rates of ethanol conversion over the commercial material. At 288°C (550°F) dealuminated H-mordenite gave 5—6 times higher rates.

The improvement in the rate of ethanol conversion resulted by partial dealumination of the commercial H-mordenite as noted in Table 3 was effected without substantial ethylene make. Table 4 provides these results.

TABLE 4
Ethylene yield (%)

$$(\% \text{ Ethylene yield} = \frac{\text{Rate of ethylene formation}}{\text{Rate of ethanol conversion}} \times 100\%)$$

| | Temperature | | |
| --- | --- | --- | --- |
| | (550°F) | (650°F) | (750°F) |
| Catalyst | (288°C) | (343°C) | (400°C) |
| 18. Silica-alumina | 0 | 0 | 4.7 |
| 19. REY Zeolite | 0 | 10 | 38 |
| 20. H—Y Zeolite | 0 | 10 | 36 |
| 21. H-Erionite | 0 | 10 | 42 |
| 22. H-Mordenite | 0 | 4 | 21 |
| 23. Partially Dealum. H-Mordenite (Ex. 1) | 0 | 4 | 21 |
| 24. Ex. 2 | 0 | 2.9 | 20 |

As noted in Table 4 dealuminated H-mordenite showed much lower ethylene make than any of the zeolite tested. This is very significant since Tables 2 and 3 show the rate of ethanol conversion over dealuminated H-mordenite is much better; e.g., 40% or better than the H—Y or rare-earth Y zeolite, and yet this is obtained with half the ethylene yield (compare Runs 18—24).

The other significant aspect is the pore shape selectivity of the dealuminated H-mordenite. When a silica-alumina, rare-earth-Y and H—Y zeolite catalyst was used the product showed triethylamine yield. On the other hand dealuminated H-mordenite showed high rates of ethanol conversion with selectivity mainly to mono and diethylamine and substantially eliminated the formation of triethylamine. It seems that the pores were large enough to make the diethylamine and allow the diethylamine to diffuse out of the pores but small enough to eliminate the formation of the triethylamine. Examples 6 and 7 of Table 5 shows traces of triethylamine formed at high temperature.

5

**0 180 983**

TABLE 5

Comparison of amine split over zeolite catalysts

|  | 288°C (550°F) | | | 343°C (650°F) | | | 400°C (750°F) | | |
|---|---|---|---|---|---|---|---|---|---|
|  | MEA | DEA | TEA | MEA | DEA | TEA | MEA | DEA | TEA |
| 1. Silica-alumina | 62.5 | 32.2 | 5.3 | 52.8 | 38.4 | 8.8 | 58.4 | 35.0 | 6.5 |
| 2. REY Zeolite | 100 | 0 | 0 | 70.5 | 27.0 | 2.50 | 78.6 | 20.2 | 1.6 |
| 3. H—Y Zeolite | 87.3 | 12.7 | 0 | 69.8 | 27.9 | 2.5 | 80.8 | 17.7 | 1.5 |
| 4. H-Erionite | t | t | t | 82.4 | 17.6 | 0 | 83.3 | 16.7 | 0 |
| 5. H-Mordenite | 100 | 0 | 0 | 89.2 | 10.8 | 0 | 86.5 | 13.5 | 0 |
| 6. Dealuminated H-Mord. (Ex. 1) | 82.3 | 17.7 | 0 | 83.5 | 16.5 | 0 | 84.4 | 15.6 | 0 |
| 7. Dealuminated H-Mord. (Ex. 2) | 87.5 | 12.5 | 0 | 81.8 | 17.9 | t | 81.5 | 18.1 | .34 |

t: trace amounts
MEA: Monoethylamine
DEA: Diethylamine
TEA: Triethylamine

Table 5 shows the significance of using dealuminated H-mordenite as compared to other catalysts in terms of amino split. Dealuminated H-mordenite has similar activity as the H—Y or rare-earth Y (REY) zeolite but it shows a slightly different amines split. Dealuminated H-mordenite showed substantially complete elimination of the triethylamine at 343°C (650°F) reaction temperature. The only catalyst that showed the preferential formation of mono and di over trisubstituted amine was H-erionite. But contrary to dealuminated H-mordenite, H-erionite showed very low rates of ethanol amination.

The elimination of the triethylamine is related to the pore size of the zeolite. H-mordenite apparently has the perfect pore size that allows the formation and movement of the mono and disubstituted amines, but not the triamine. The Y zeolite has large pores to allow the formation of the triethylamine. By dealuminating the H-mordenite, it becomes clear that the pore shape selectivity inherent in the H-mordenite has not been modified.

**Claims**

1. Vapor phase catalytic amination process for the production of amines by reacting a reaction mixture comprising an $C_{2-8}$ alkanol or $C_{2-6}$ alkyl ether and a nitrogen compound selected from the group consisting of ammonia and primary and secondary amines at a temperature and pressure sufficient for effecting formation of said amine, and in the presence of a crystalline alumino silicate as a catalyst, characterized in that as the catalyst a dealuminated crystalline alumino-silicate zeolite of the molecular sieve type is used.

2. The process of Claim 1 wherein said dealuminated crystalline alumino-silicate has sufficient aluminum atoms removed to reduce the weight of alumina 10—90% by weight of the total alumina in the zeolite.

3. The process of Claim 2 wherein the reaction mixture comprises a $C_{2-4}$ alkanol and the nitrogen compound is ammonia.

4. The process of Claim 3 where said crystalline alumino-silicate which has been dealuminated is a Y zeolite or mordenite.

5. The process of Claim 4 wherein said dealuminated Y zeolite or mordenite is ion exchanged with a trivalent rare earth metal ion or hydrogen ion.

6. The process of Claim 5 wherein the Si to Al mole ratio is from 6—100:1 for said mordenite and 2.5—15:1 for said Y zeolite.

7. The process of Claim 5 wherein the reaction mixture comprises ethanol and the mole ratio of ammonia to ethanol is from about 1—10:1.

8. The process of Claim 7 wherein the pressure utilized in carrying out the amination is from $6,89 \times 10^5$ to $1,37 \times 10^7$ Pa (100—2000 psig) and the gas hourly space velocity is 500—12000 hrs$^{-1}$.

**0 180 983**

**Patentansprüche**

1. Dampfphasenkatalytisches-Aminierungsverfahren zur Herstellung von Aminen durch Umsetzen einer Reaktionsmischung, enthaltend einen $C_{2-8}$ Alkanol oder $C_{2-6}$ Alkylether und eine Stickstoffverbindung, die aus der Gruppe Ammoniak und primäre und sekundäre Amine ausgewählt ist, bei einer Temperatur und einem Druck, die zur Bildung der Amine ausreichend sind und in Gegenwart eines kristallinen Aluminiumsilikats als Katalysator, dadurch gekennzeichnet, daß als Katalysator ein dealuminierter kristalliner Aluminosilikatzeolit vom Molekularsieb-Typ verwendet wird.

2. Verfahren nach Anspruch 1, worin im dealuminierten kristallinen Aluminiumsilikat genügend Aluminiumatome entfernt wurden, um das Gewicht von Aluminium um 10 bis 90 Gew.-% des gesamten Aluminiums im Zeolit zu reduzieren.

3. Verfahren nach Anspruch 2, worin die Reaktionsmischung einen $C_{2-4}$ Alkanol enthält und die Stickstoffverbindung Ammoniak ist.

4. Verfahren nach Anspruch 3, worin das kristalline Aluminiumsilikat, das dealuminiert wurde, ein Y-Zeolit oder Mordenit ist.

5. Verfahren nach Anspruch 4, worin der dealuminerte Y-Zeolite oder Mordenit mit einem trivalenten Seltenerdmetallion oder Wasserstoffion Ionen ausgetauscht wird.

6. Verfahren nach Anspruch 5, worin das Molverhältnis von Si zu Al 6 bis 100:1 für den Mordenit und 2,5 bis 15:1 für den Y-Zeolit beträgt.

7. Verfahren nach Anspruch 5, worin die Reaktionsmischung Ethanol enthält und das Molverhältnis von Ammoniak zu Ethanol etwa 1 bis 10:1 beträgt.

8. Verfahren nach Anspruch 7, worin der in der Durchführung der Aminierung verwendete Druck $6,9 \times 10^5$ bis $1,37 \times 10^7$ Pa (100 bis 2000 psig) beträgt und der Gasfluß pro Stunde (gas hourly space velocity) 500 bis 12000 $h^{-1}$ beträgt.

**Revendications**

1. Procédé d'amination catalytique en phase vapeur pour la production d'amines en faisant réagir un mélange réactionnel comprenant un alcanol en $C_2$—$C_8$ ou un éther alkylique en $C_2$—$C_6$ et un composé d'azote choisi parmi le groupe comprenant l'ammoniac, ainsi que les amines primaires et secondaires, à une température et sous une pression suffisantes pour effectuer la formation de cette amine, ainsi qu'en présence d'un aluminosilicate cristallin comme catalyseur, caractérisé en ce que, comme catalyseur, on utilise une zéolite d'alumino-silicate cristalline désaluminée du type des tamis moléculaires.

2. Procédé selon la revendication 1, caractérisé en ce qu'une quantité suffisante d'atomes d'aluminium est éliminée de l'aluminosilicate cristallin désaluminé afin de réduire le poids de l'alumine de 10—90% en poids de la quantité totale d'alumine contenue dans la zéolite.

3. Procédé selon la revendication 2, caractérisé en ce que le mélange réactionnel comprend un alcanol en $C_2$—$C_4$, tandis que le composé azoté est l'ammoniac.

4. Procédé selon la revendication 3, caractérisé en ce que l'alumino-silicate cristallin qui a été désaluminé est une mordénite ou une zéolite Y.

5. Procédé selon la revendication 4, caractérisé en ce que la mordénite ou la zéolite Y désaluminée a subi un échange d'ions avec un ion hydrogène ou un ion d'un métal de terres rares trivalent.

6. Procédé selon la revendication 5, caractérisé en ce que le rapport molaire Si:Al se situe entre 6—100:1 pour la mordénite et entre 2,5—15:1 pour la zéolite Y.

7. Procédé selon la revendication 5, caractérisé en ce que le mélange réactionnel comprend de l'éthanol, tandis que le rapport molaire entre l'ammoniac et l'éthanol se situe entre environ 1 et 10:1.

8. Procédé selon la revendication 7, caractérisé en ce que la pression utilisée pour effectuer l'amination se situe entre $6,89 \times 10^5$ et $1,37 \times 10^7$ Pa (100—2,000 livres/pouce carré manométrique), tandis que la vitesse spatiale horaire de gaz est de 500—12,000 $h^{-1}$.